# EUROPÄISCHE PATENTSCHRIFT

(11) **EP 4 561 471 B1**
(45) Veröffentlichungstag und Bekanntmachung des Hinweises auf die Patenterteilung: **27.05.2026**
(21) Anmeldenummer: 23728740.4
(22) Anmeldetag: 24.05.2023
(51) Int. Cl.: A61B 17/70

(54) **INSTRUMENTENSATZ UND TULPE SELBIGENS**
INSTRUMENT SET AND TULIP OF THE SAME
ENSEMBLE D'INSTRUMENTS ET TULIPE CORRESPONDANTE

(30) Priorität: 29.07.2022 DE 102022002763
(43) Veröffentlichungstag der Anmeldung: 04.06.2025
(73) Patentinhaber: Taurus GmbH & Co. KG, 63755 Alzenau (DE)
(72) Erfinder: SIEDLER, Uwe, 63755 Alzenau (DE); HEUER, Frank, 63755 Alzenau (DE)
(74) Vertreter: Leinweber & Zimmermann
(86) Internationale Anmeldenummer: PCT/EP2023/063961
(87) Internationale Veröffentlichungsnummer: WO 2024/022642

(56) Entgegenhaltungen:
- EP-A1- 2 792 325
- EP-A1- 2 957 246
- DE-A1- 102015 205 362
- DE-A1- 102020 005 928
- US-A1- 2003 199 872
- US-A1- 2014 277 137

## Beschreibung

Die vorliegende Erfindung betrifft das Gebiet der Wirbelsäulenimplantate, insbesondere derartiger Implantatsysteme, die Pedikelschrauben aufweisen, von denen mehrere über eine Verbindungsstange bzw. einen Fixierstab zur Stabilisierung der Wirbelsäule miteinander verbunden werden. Insbesondere betrifft die Erfindung einen Instrumentensatz aufweisend eine sich entlang ihrer Längsachse erstreckende Tulpe eines eine Pedikelschraube aufweisenden und der Ankopplung einer Verbindungsstange entlang einer Querachse der Tulpe dienenden Wirbelsäulenimplantats sowie eine Haltevorrichtung zum temporären Halten der Tulpe während eines Implantateinsatzvorgangs in einem Haltezustand des Instrumentensatzes, wobei ein Übergang vom Haltezustand in einen Freigabezustand von Tulpe und Haltevorrichtung und vice versa eine rotatorische Bewegung um eine Achse beinhaltet.

Derartige Implantatsysteme sind dem Fachmann selbstverständlich wohlvertraut, beispielhaft wird auf die US 2012/0041490 A1 oder EP 2 376 005 B1 verwiesen. Solche Haltesysteme wie in dieser und zahlreichen anderen Techniken, die auf geraden (rein tangentialen) Nuten in den Tulpenwand-Außenseiten beruhen und damit ein tangentiales Aufschieben eines Halteinstruments erlauben, jedoch kein rotatorisches Eindrehen eines Halteinstruments um die Tulpenlängsachse, wurden inzwischen weiterentwickelt zu Haltesystemen, bei denen ein Haltezustand zwischen einer Haltevorrichtung und der Tulpe über eine Verdrehung des Halteinstruments um die Längsachse der Tulpe erreicht wird. Dies ist beispielsweise in US 9,050,148 B2 offenbart, mit einer der zylindrischen Tulpenseitenaußenwand in umfänglicher Richtung folgenden und zur Seite hin offenen Nut.

Der Erfindung liegt die Aufgabe zugrunde, einen Instrumentensatz der eingangs genannten Art nochmals zu verbessern, insbesondere vor dem Hintergrund der Kombination einer einfachen Bedienbarkeit bei zuverlässigem Übergang von Freigabe in den Haltezustand.

EP 2 957 246 A1 offenbart ein System aus Polyaxialschraube, Tulpe und Bedieninstrument, bei dem eine Innenhülse des Bedieninstruments leicht flexibel ist und durch eine Axialbewegung auf die Tulpe aufgeklipst werden kann. US 2003/0199872 A1 offenbart ein in ähnlicher Weise wirkendes Aufklipsen mittels eines Bedienelements mit pistolenartigem Griff. DE 10 2020 005 928 A1 offenbart einen zusätzlichen Verriegelungsring für die Tulpe, der nicht nur über Stabauflagestellen, sondern auch über einen Vorsprung betätigt werden kann. US 2014/0277137 A1 lehrt eine Unterstützung der Einführbewegung für eine Kopplung von Tulpe und Instrument in unteren Beinbereichen durch ein Zusammendrücken von oberen Beinbereichen des Instruments, und eine geeignet dünne Kopplung zwischen den Beinbereichen. DE 10 2015 205362 A1 offenbart schwenkbare Klammerschenkel des Bedieninstruments, wobei die zugehörige Schwenkachse orthogonal zur Querrichtung verläuft, in der die Tulpen mehrere Implantate verbindende Korrekturstab eingelegt wird. EP 2 792 325 A1 offenbart einen Instrumentensatz gemäß dem Oberbegriff von Anspruch 1.

Die der Erfindung zugrundeliegende Aufgabe wird durch einen Instrumentensatz mit allen Merkmalen von Anspruch 1 gelöst.

Hierbei ist einerseits vorgesehen,
dass die Achse mit überwiegender Richtungskomponente sowohl quer zur Längsachse als auch parallel zur Querachse der Tulpe verläuft. In Kugelkoordinaten mit der Tulpenlängsachse als z-Achse und der Tulpenquerachse als x-Achse gilt somit ϑ > 45° und φ < 45°. Bevorzugt liegen diese Winkel jedoch näher an 90° (ϑ) bzw. an 0° (φ), bevorzugt nicht mehr als 30° von diesen Werten entfernt, weiter bevorzugt nicht mehr als 20°, insbesondere nicht mehr als 10°. In einer bevorzugten Ausgestaltung verläuft die Achse der rotatorischen Bewegung im Wesentlichen parallel zur Querachse, wenn sich Tulpe und Halteinstrument im Haltezustand befinden. Die Achse der rotatorischen Bewegung ist eine definierte Achse, die durch das Halteinstrument definiert ist und im System des Halteinstruments raumfest ist.

Mit der erfindungsgemäßen Lösung ist eine Verdrehbarkeit von Halteinstrument und Tulpe um die Tulpenlängsachse nicht mehr erforderlich, so dass während des Übergangs eine zuverlässige Azimutalzuordnung zwischen Halteinstrument und Tulpe gegeben ist.

Weitere vorteilhafte Ausführungsformen sind in den abhängigen Ansprüchen angegeben.

So ist bevorzugt vorgesehen, dass bei dem Instrumentensatz innerhalb des Übergangs eine Transition zwischen einer bei bezüglich der Tulpenlängsachse radial auf die Tulpe einwirkender Haltekraft bereits wirkenden Verdrehsperre gegen eine Verdrehung der Haltevorrichtung gegenüber der Tulpe um deren Längsachse und einer die rotatorische Bewegung nach bezüglich der Tulpenlängsachse radial außen sperrenden Rotationssperre erfolgt. Die Verdrehsperre gegen Verdrehung um die Tulpenlängsachse ist somit beim Übergang vom Freigabezustand zum Haltezustand nicht erst im finalen Haltezustand gegeben, sondern bereits in einem Zwischenhaltezustand. Durch die rotatorische Bewegung bei der Schließbewegung auf die Tulpe einwirkenden Kräfte gleichen sich hinsichtlich ihrer Tangentialkomponenten im Wesentlichen aus, so dass effektiv radial einwärts gerichtete Kräfte wirken.

Weiter bevorzugt ist vorgesehen, dass der Übergang des Instrumentensatzes jedenfalls haltezustandsnah eine translatorische Axialbewegung zwischen Tulpe und Haltevorrichtung entlang der Tulpenlängsachse beinhaltet. Dies erhöht die axiale Tiefe der Rotationssperre.

Weiterhin ist bevorzugt vorgesehen, dass bei dem Instrumentensatz die rotatorische Bewegung und die Axialbewegung in dem Übergang wenigstens zeitweise überlappen. Hierzu kann eine Führung vorgesehen werden, die bevorzugt durch zueinander passende Formgestaltung, etwa durch geeignete Schrägen oder Rundungen der jeweiligen Kopplungsbereiche realisiert ist, etwa an der Unterseite eines Radialvorsprungs des Instruments, und eine radial nach innen aufwärts gerichtete Fläche der Tulpe.

Ebenfalls bereitgestellt wird eine Tulpe eines Instrumentensatzes gemäß der vorliegenden Erfindung. Erfindungsgemäß weist die Tulpe wenigstens vier azimutal voneinander beabstandete tulpenseitige Kopplungsbereiche zur Ankopplung der Haltevorrichtung im Haltezustand auf. Diese sind gegeneinander bezüglich der Querachse bevorzugt achsensymmetrisch gestaltet und realisieren als eine bevorzugte Variante die oben erwähnte Kraftaufteilung und -kompensation.

Weiter weist ein Kopplungsbereich der Tulpe eine Einkerbung mit insbesondere konkav verlaufendem Boden zur Bildung der Verdrehsperre gegenüber einer Verdrehung des Instruments um die Längsachse auf. "Konkav" bzw. "konkave Grundform" bedeutet im Sinne dieser Anmeldung keine perfekte mathematische Konkavität, sondern eine Abgrenzung von rein geraden (tangentialen) Verlaufsformen sowie den anders herum gekrümmten konvexen Grundformen. Definiert etwa eine Kreislinie um eine Sekante ein Krümmungsvorzeichen, so bedeutet die Konkavität eine über den Verlauf des Kerbenbodens gemittelte entgegengesetzte Krümmung.

Auch weist der Kopplungsbereich der Tulpe einen axial an die Azimutalkerbe anschließenden unteren (proximalen) Axialkanalbereich auf, für den tulpenseitigen Rotationssperrenbereich der Rotationssperre.

Bevorzugt weist die Tulpe mehrere in die obere Axialseite der Tulpenseitenwände mündende obere Axialkanalbereiche auf, welche insbesondere mit den unteren Kanalbereichen kommunizieren und mit ihnen fluchten. Die oberen Axialkanalbereiche stellen zwar eine an sich ungewünschte Materialschwächung der Tulpenseitenwände dar, werden jedoch aus herstellungstechnischen Gründen bevorzugt.

In einer Ausführungsform verlaufen die Axialkanalbereiche im Querschnitt orthogonal zur Längsachse gesehen linear und gegenüber der Längsachse bevorzugt tangential. Sie könnten in einer andern Ausführungsform jedoch in diesem Querschnitt auch polygonal verlaufen. Ebenfalls bevorzugt wird eine Variante, bei der dieser Verlauf gekrümmt ist, mit bevorzugt einem Krümmungsmittelpunkt im wesentlichen im Zentrum der Tulpe (d.h. beim Durchstoßpunkt der Längsachse durch die Querschnittsebene). Dies gilt für untere und/obere Axialkanalbereiche.

Weiterhin bevorzugt weist die Tulpe zum axialen distalen Tulpenende weisende Seitenwände der Azimutalkerben auf, die einen in einer Radialebene liegenden planaren Flächenabschnitt aufweisen oder gänzlich in einer Radialebene liegen, insbesondere auf jeweilig gleicher axialer Höhe. Bevorzugt hat eine Seitenwand der Tulpe wenigstens zwei solche Azimutalkerben.

Noch weiter bevorzugt weist die Tulpe eine zur proximalen axialen Endseite der Tulpe weisende Seitenwand der Azimutalkerbe auf, die rampenförmig gebildet ist. Die rampenförmige Gestaltung erleichtert den Übergang zwischen rotatorischer und translatorischer Bewegung und schafft die erforderlichen Freiräume für die Einführung des halteinstrumentseitigen Anteils der Rotationssperre. In einer Ausführungsform stehen einander zugewandte planare Flächenabschnittsbereiche im jeweiligen Boden zweier in derselben Seitenwand angeordneten Azimutalkerben miteinander unter einem von Null verschiedenen Winkel, bevorzugt einem Winkel von wenigstens 20 Grad.

Bei einer weiteren bevorzugten Ausgestaltung weist die Tulpe einen azimutal zwischen zwei an einer Seitenwand der Tulpe ausgebildeten Azimutalkerben gebildeten Stegbereich auf, insbesondere mit radial nach außen abnehmender Azimutalabmessung. Bevorzugt beträgt der Anteil der Axialkanalbereiche in Umfangsrichtung gesehen nicht mehr als 70% des Azimutalbereichs der Seitenwand, bevorzugt nicht mehr als 65%, insbesondere nicht mehr als 60%. Die azimutale Abmessung des Stegbereichs am Stegfuß, also auf radialer Höhe der Kerbenböden und axialer Höhe der proximalen Kerbenseitenfläche beträgt bevorzugt wenigstens 12°, bevorzugt wenigstens 18°, insbesondere wenigstens 24°.

Zwei in jeweils einer anderen Seitenwand angeordnete Azimutalkerben können jeweils einen parallel zueinander verlaufenden planare Flächenabschnitt aufweisen. Bevorzugt ist eine Azimutalkerbe in einer Seitenwand diametral entgegengesetzt zu einer Azimutalkerbe einer anderen Seitenwand angeordnet.

Bevorzugt übersteigt eine axiale Länge eines distal der Azimutalkerben angeordneten axiale Endbereichs der Tuple und/oder die der Axialkanäle nicht das 1,6-fache der axialen Abmessung des Kerbenbodens an seiner radial innersten Stelle, weiter bevorzugt nicht deren 1,3-faches, insbesondere nicht deren 1,1-faches. Dies schafft einen axial kompakten Aufbau der Tulpe. Hinsichtlich der letzteren beiden Werte besteht dieses Verhältnis bevorzugt auch bezüglich der axialen Abmessung der Kerbe an ihrer radial äußersten Stelle.

Ebenfalls bereitgestellt wird ein Verfahren zur Herstellung einer Tulpe, bei der die Ausbildung des unteren Axialkanalbereichs nach der Ausbildung des oberen Kanalbereichs erfolgt, und bei der die Ausbildung des unteren Kanalbereichs insbesondere vor der Ausbildung der Azimutalkerbe erfolgt. Auf diese Weise wird die Gefahr einer Beschädigung der durchaus filigranen Tulpenseitenwände reduziert. Das Halteinstrument weist bevorzugt zwei durch Rotation um die Achse der rotatorischen Bewegung verschwenkbare Beine auf, an deren Radialinnenseite jeweils passend zur Anzahl der Kopplungsbereiche an der Tulpe die halteinstrumentseitigen Kupplungsbereiche ausgebildet sind. Letztere sind bevorzugt aus einem Radialvorsprung gebildet, auf dem jeweils ein Axialvorsprung angeflanscht ist. Der Radialvorsprung greift jeweils in die Azimutalkerbe der Tulpe ein, der Axialvorsprung in den unteren Axialkanal.

Bevorzugt sind die Beine des Halteinstruments zum Erreichen einer Freigabestellung vorgespannt, etwa durch eine elastische Einrichtung. Des Weiteren weist das Halteinstrument bevorzugt einen Verriegelungsmechanismus auf, mit dem es gegen die Vorspannung in einen dem Haltezustand entsprechenden Schließzustand des Halteinstruments verriegelbar ist. Es versteht sich, dass über die Verriegelung ebenfalls eine (erste) Rotationssperre für eine Relativverschwenkbewegung der Beine voneinander weg gebildet ist. Nach Erkenntnis der vorliegenden Erfindung ist diese Rotationssperre jedoch in Einzelfällen nicht ausreichend, so dass durch die über die Kopplungsbereiche des Halteinstruments und Tulpe bereitgestellte Rotationssperre eine zusätzliche Sicherheit gegen eine solche öffnende Relativbewegung der Beine gegeben ist.

Weitere Einzelheiten, Merkmale und Vorteile der Erfindung ergeben sich aus der nachfolgenden Beschreibung mit Bezug zu den beiliegenden Figuren, von denen
Fig. 1 einen Instrumentensatz mit einer Tulpe eines Wirbelsäulenimplantats und einen unteren Teil eines Halteinstruments zeigt,
Fig. 2 den Instrumentensatz aus Fig. 1 links in einer Zwischenstellung zum Erreichen des Haltezustands nebst Detailansicht und rechts in einem Haltezustand zeigt,
Fig. 3 das Wirbelsäulenimplantat aus Fig. 1 nebst vergrößerter Teilansicht des oberen Bereichs mit der Tulpe zeigt,
Fig. 4 eine Seitenansicht des Wirbelsäulenimplantats nebst zwei Radialschnittansichten unterschiedlicher axialer Höhe zeigt,
Fig. 5 eine Fig. 4 entsprechende Ansicht einer anderen Ausführungsform zeigt,
Fig. 6 eine Fig. 4 entsprechende Ansicht einer nochmals anderen Ausführungsform zeigt,
Fig. 7 das Halteinstrument aus Fig. 1 in einer perspektivischen Ansicht vollständig zeigt, in einem geöffneten Zustand, und
Fig. 8 dieses Halteinstrument in einem geschlossenen, dem Haltezustand in Fig. 2, rechts, entsprechenden Zustand zeigt.

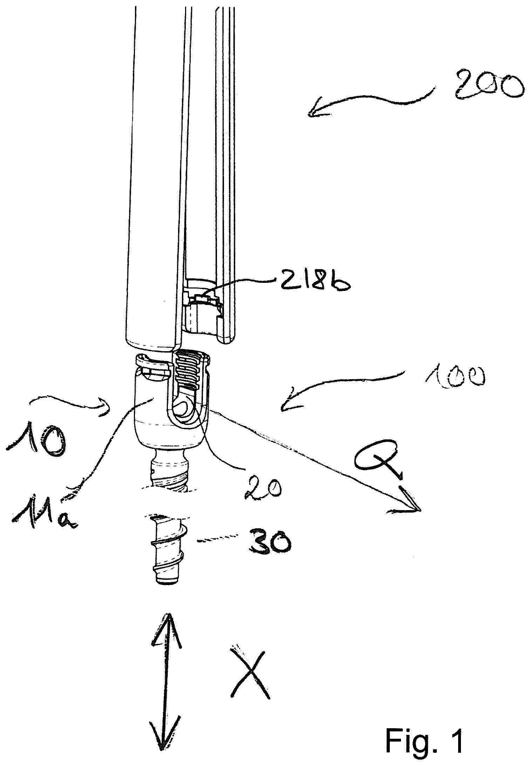

Die Grundfunktionalität des in Fig. 1 dargestellten Wirbelsäulenimplantats 100 ist dem Fachmann wohl vertraut. Es weist einen Knochenanker in Form einer Pedikelschraube 30 auf, welche in dem üblicherweise als Tulpe 10 bezeichneten Kopf des Implantats 100 schwenkbar, insbesondere polyaxial schwenkbar, gegenüber der Längsachse X der Tulpe 10 gelagert ist. Der Grundaufbau der Tulpe 10 ist ebenfalls typisch und dem Fachmann wohl bekannt, neben einem Aufnahmeraum im unteren Bereich zur Aufnahme des (verdeckten) Kopfes der Pedikelschraube 30 ist ein oberer, von der äußeren Grundform her zylindrischer Umrandungsbereich zur Bildung einer in Querrichtung Q verlaufenden Aufnahmerinne für eine (nicht dargestellte) Verbindungsstange vorgesehen, mit welcher eine Pedikelschraube mit einer weiteren Pedikelschraube gekoppelt werden kann.

Im oberen Bereich verbleiben somit zwei Seitenwände 11a, 11b der Tulpe 10, an deren Innenseite ein Gewinde zur Aufnahme einer (ebenfalls nicht dargestellten) Setzschraube (etwa einer Madenschraube) gebildet ist, mit welcher die Verbindungsstange gegenüber der Tulpe 10 fixierbar ist. Mit dem Festschrauben der Setzschraube wird zudem auch die Lage der Pedikelschraube 30 gegenüber der Tulpe 10 fixiert, in dem gezeigten Ausführungsbeispiel nicht durch unmittelbaren kraftübertragenden Kontakt der Verbindungsstange auf den Schraubenkopf, sondern durch eine indirekte Kraftübertragung mittels eines üblicherweise als Sattel 20 bezeichneten Zwischenstücks, welches in seinem unteren Bereich den Schraubenkopf wenigstens teilweise aufnimmt und an seiner Oberseite passend zur Anlage an die in Querrichtung Q verlaufenden Verbindungsstange geformt ist.

Es versteht sich, dass die Erfindung hinsichtlich den zahlreichen Gestaltungsmöglichkeiten der bislang beschriebenen Grundfunktionalität nicht eingeschränkt ist, etwa hinsichtlich konkreter Gestaltungen von Sattel 20, Innengestaltung der Tulpe 10, Monoaxialität oder Polyaxialität, Symmetrie oder Asymmetrie der für das Verschwenken gegenüber der Längsachse (Axialachse) X verfügbaren Raumwinkelbereiche, Materialauswahl des Implantats, Gewindegestaltung der Pedikelschraube 30 und/oder etwaigen Zusatzfunktionalitäten keinerlei besonderen Einschränkungen unterworfen ist.

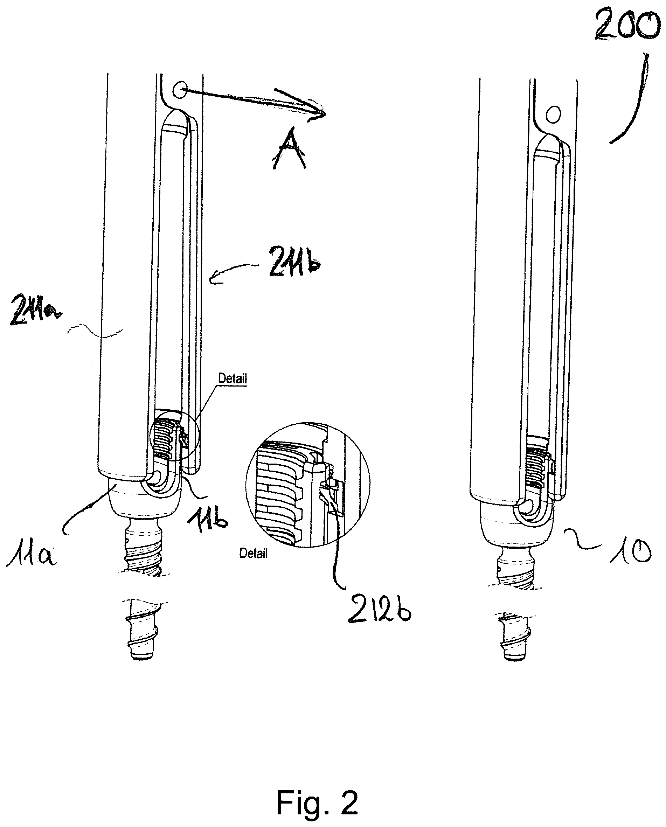

Zum Halten der Tulpe 10 beim Einsetzen des Implantats 100, insbesondere im minimalinvasiven Verfahren, ist ein Halteinstrument 200 vorgesehen. Wie besser aus dem in Fig. 2, rechts, gezeigten Haltezustand zwischen Instrument 200 und Tulpe 10 erkennbar ist, weist das Halteinstrument 200 zwei Beine 211a und 211b auf, die jeweils mit einer der Seitenwände 11a, 11b gekoppelt werden. Obgleich der Übergang von einem Freigabezustand in diesen Haltezustand eine rotatorische Bewegung um eine Achse beinhaltet, stimmt diese Achse nicht mit der herkömmlichen rotatorischen Achse zusammen, welche im Stand der Technik durch die Längsachse X der Tulpe gegeben ist. Vielmehr verläuft die Achse A der rotatorischen Bewegung in Querrichtung Q. Das Instrument wird somit nicht um die Axialachse X gegenüber der Tulpe verdreht, sondern die Kopplungsbewegung umfasst eine durch die rotatorische Bewegung hervorgerufene Verschwenkung der Beine 211a, 211b gegeneinander, unter entsprechender Annäherung an die Tulpe 10 in bezüglich der Axialachse X überwiegend radialer und orthogonal zur im Bezugssystem des Instruments raumfest definierten Achse A der rotatorischen Bewegung verlaufenden Richtung.

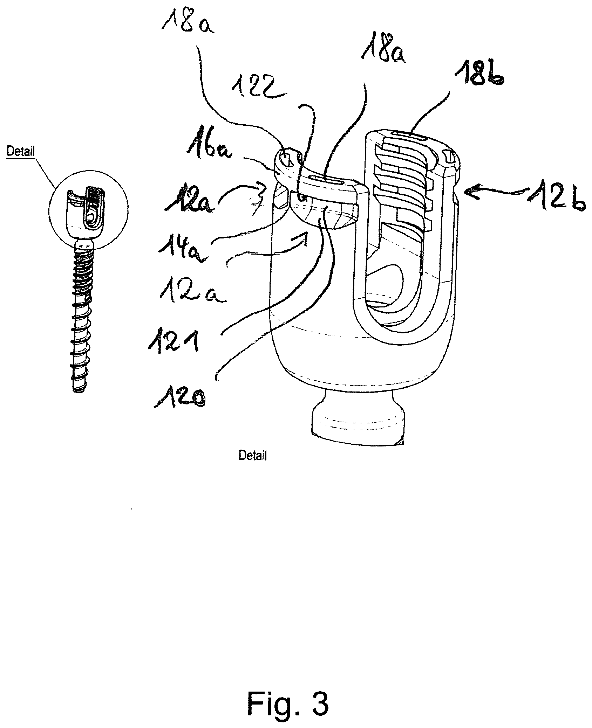

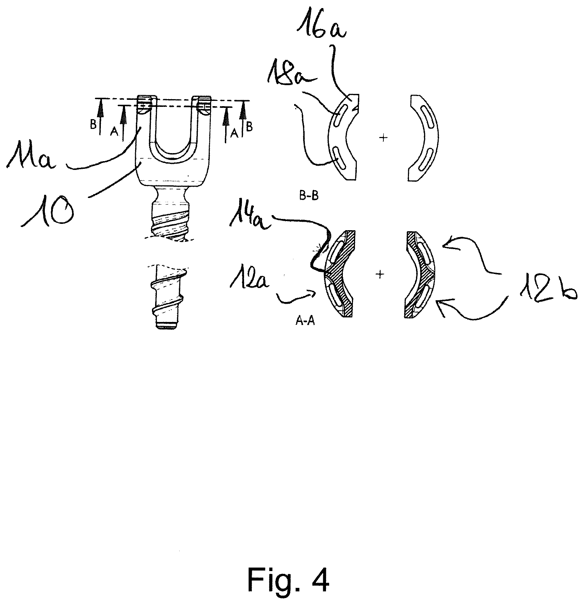

Tulpenseitliche Kopplungsbereiche 12a, 12b an den Seitenwänden 11a, 11b sind in diesem Ausführungsbeispiel an beiden Seiten gleich ausgebildet, so dass sich die nachfolgende Beschreibung auf nur eine Seite beschränkt. Wie am besten in Fig. 3 erkennbar ist, weist die Tulpe 10 nahe ihrer oberen axialen Endseite von der Grundform her konkav geformte, als Kopplungsbereiche dienende Einschnitte 12a, 12a auf, die somit gerade nicht der Umfangsrichtung der konvexen, im Wesentlichen zylindrischen Seitenwand 11a folgen, sondern eine Drehsperre für die instrumentenseitigen Gegenkopplungsstücke gegen eine Verdrehung des Instruments 200 bezüglich der Tulpe 10 um deren Längsachse X bilden. Die Gegenstücke gelangen somit bereits in einem Zwischenhaltezustand vor Erreichen des finalen Halteendzustands gegen eine solche Verdrehkraft weder in die konkaven kerbenartigen Kopplungsbereiche 12a hinein, noch aus ihnen hinaus. Dies ist am besten in der Radialschnittansicht A-A in Fig. 4 zu erkennen.

Der konkave, kerbenartige Kopplungsbereich 12a, im Folgenden auch kurz als Kerbe 12a bezeichnet, weist in einer auf die Radialrichtung bezüglich der Axialachse X bezogenen Nomenklatur eine Bodenfläche 120, eine erste Seitenfläche 121 und eine zweite Seitenfläche 122 auf. Die erste Seitenfläche 121 folgt an ihrer an die Bodenfläche 120 angrenzenden Seite deren Verlauf, und an ihrem entgegengesetzten Rand dem Verlauf der konvexen Seitenwand 11a. Sie verläuft jedoch nicht in der Radialebene, sondern ihr Normalenvektor, der über den Verlauf der ersten Seitenfläche 121 keinesfalls konstant sein muss, weist insbesondere nahe der Bodenfläche 120 eine radial nach außen weisende Richtungskomponente auf.

Die zweite Seitenfläche 122 ist dagegen in diesem Ausführungsbeispiel im Wesentlichen planar gebildet und erstreckt sich somit bezüglich der Axialachse X in einer Radialebene. Der axiale Abstand zwischen der zweiten Seitenfläche 122 und der ersten Seitenfläche 121 ist somit jedenfalls in einem azimutalen Mittenbereich am Übergang zur konvexen Außenfläche der Seitenwand 11a größer als bodenseitig. In der in den Figuren 1 bis 4 dargestellten Ausführungsform ist speziell vorgesehen, dass dieser axiale Abstand von radial innen nach radial außen ansteigt, und auch azimutal von außen in Richtung auf die azimutale Mitte der Kerbe 12a ansteigt. Die Kerben 12a sind azimutal beabstandet, und der azimutale Abstand ihrer azimutalen Mitten beträgt in diesem Ausführungsbeispiel ca. 60°.

Die in diesem Ausführungsbeispiel zwei Kerben 12a sind durch einen Steg 14a voneinander getrennt. Aufgrund der konkaven Gestaltung der Bodenfläche 120 der Kerben 12a weist der Steg 14a im Radialschnitt eine im Wesentlichen trapezartige, fast dreieckartige Form auf, also einer radial innen im Vergleich zu radial außen deutlich höheren Azimutalabmessung, die in diesem Ausführungsbeispiel ca. 30° beträgt. Diese Gestalt dient insbesondere der Schaffung einer größeren Materialsteifheit zur Kompensation einer Materialschwächung, welche die Tulpenwand 11a in dem in Axialrichtung X zwischen den Kerben 12a und dem oberen axialen Ende der Tulpe 10 gelegenen Bereich aufweist, die am besten in den Figuren 3 und insbesondere dem Radialschnitt B-B von Fig. 4 zu erkennen ist.

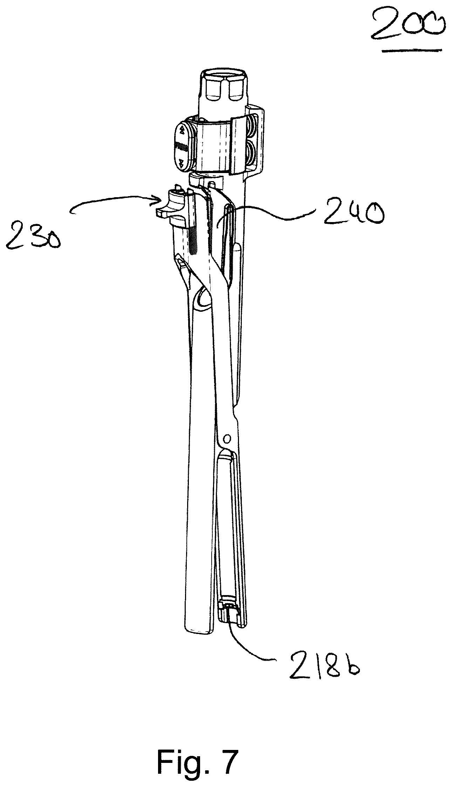

So weist die Tulpe 10 in diesem Bereich 16a zwei in Axialrichtung X verlaufende Bohrungen 18a auf, die azimutal als Langlöcher ausgebildet sind, deren Azimutalerstreckung sich nur über einen Teil, in dieser Ausführungsform etwa die Hälfte der Azimutalerstreckung der Kerben 12a erstreckt, und deren in Radialrichtung innere Innenwand axial im Wesentlichen mit der Bodenfläche 120 der Kerbe 12a fluchtet. In dem Langloch 18a ist im Haltezustand ein in Fig. 1 bzw. Fig. 2 aufgrund Verdeckung durch das Instrumentenbein 211a nicht erkennbarer Axialvorsprung 218a aufgenommen, erkennbar in Fig. 7 ist jedoch der auf dem in Fig. 1 erkennbaren Radialvorsprung 212b angeordnete Axialvorsprung 218b zur Aufnahme im Langloch 18b der gegenüberliegenden Seitenwand 11b. Die radial äußere Begrenzung des Langlochs 18a (18b) bildet damit im Haltezustand eine Rotationssperre gegen ein Verschwenken des Beins 211a um die Achse A der rotatorischen Bewegung des Instruments 200.

Die Langlöcher 18a, 18b sind azimutal beidseits geschlossen, in dieser Ausführungsform ist das Verhältnis aus nicht von den Langlöchern 18a, 18b belegten und somit von Material der Tulpe eingenommenen Materialbereich in Azimutalrichtung zur Gesamt-Azimutalerstreckung der Seitenwand 11a der Tulpe im radialen Bereich der Langlöcher 18a, 18a ca. 50%.

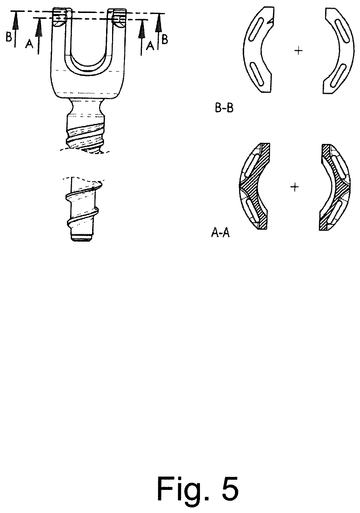

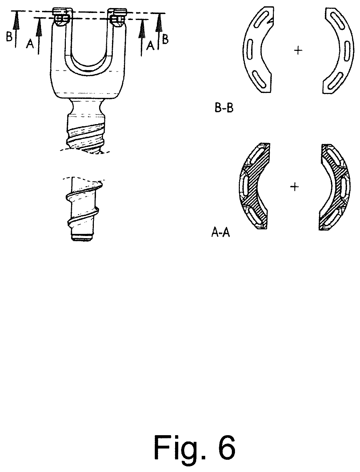

In der Ausführungsform von Fig. 5 ist der Verlauf der Langlöcher 18a, 18b nicht konzentrisch wie in der Ausführungsform von Fig. 4, sondern tangential, die Langlöcher sind somit gerade ausgebildet. In der Ausführungsform gemäß Fig. 6 sind jeweils drei Langlöcher pro Seitenwand 11a, 11b vorgesehen, hier mit konzentrischer Erstreckung wie in der Ausführungsform von Fig. 4, auch diese können jedoch ähnlich wie in der Ausführungsform gemäß Fig. 5 in einer weiteren, nicht dargestellten Ausführungsform gerade (tangential) gestaltet sein.

Wie durch Fig. 2 veranschaulicht ist, wird der Haltezustand in Fig. 2 rechts dadurch erreicht, dass die Beine 211a, 211b durch Ausführung der Schwenkbewegung um die Achse A radial einwärts von den Innenseiten der Beine 211a, 211b vorragende Radialvorsprünge 212a, 212b zunächst überwiegend radial in die ihnen zugewandten Kerben 12a, 12b verbringen. Der finale Haltezustand wird durch eine axiale Translationsbewegung zwischen Instrument 200 und Tulpe 10 erreicht, wobei sich die rotatorische Bewegung um die Achse A und diese translatorische Bewegung bereits überlappen können. In den in den Figuren gezeigten Ausführungsbeispielen bilden die erste Seitenfläche 121 und die dieser Seitenfläche 121 zugewandte untere Fläche des Radialvorsprungs 212a, b eine eine solche translatorische Axialbewegung aus der rotatorischen Bewegung heraus unterstützende und insbesondere ggf. auch generierende Zwangsführung. Entsprechend kann vice versa beim Lösen von Instrument 200 und Tulpe 10 eine translatorische Axialbewegung entlang der Längsachse X der Tulpe eingeleitet werden, und die rotatorische Bewegung kann von dieser Zwangsführung geführt bzw. eingeleitet werden.

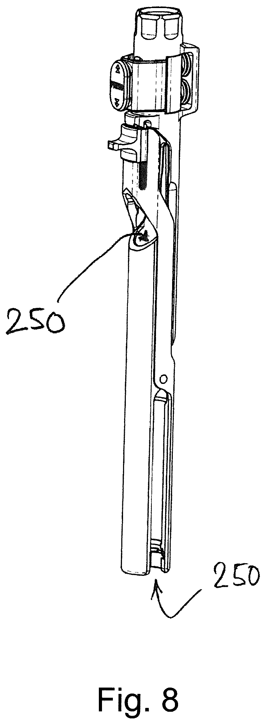

In den Figuren 7 und 8 ist das Halteinstrument 200 nochmals ganzteilig abgebildet. In dem in Fig. 7 gezeigten Öffnungszustand sind die Beine 211a, 211b aus ihrer parallelen Grundkonfiguration (Fig. 8) im Haltezustand gegeneinander verschwenkt. Ein Schiebeschalter 230, der an einem Fortsatz des Beins 211b jenseits der Achse A angeordnet ist, kann den Haltezustand (Fig. 8) gegenüber der Rückstellkraft einer federnden Einrichtung 240 erhalten. Im Haltezustand der Tulpe wird der Verriegelungsmechanismus des Schalters 230 jedoch von an den freien Enden der Beine 211a, 211b angreifenden, insbesondere radial nach außen wirkenden Kräften nicht mehr beansprucht, da diese von der durch die Kopplungsbereiche der Tulpe 10 gebildeten Rotationssperre aufgenommen werden.

Der zwischen den Beinen 211a, 211b gebildete Innenkanal 250 setzt sich zum distalen Ende fort, so dass innerhalb des Instruments ein (nicht dargestellter) innengeführter Stempel geführt werden kann, um die oben erläuterte Verbindungsstange (auch Fixierstab genannt) in die Tulpe zu drücken. Der in Querrichtung zwischen den Beinen 211a, 211b des Instruments 200 bestehende Freibereich ist hierzu auch ausreichend dimensioniert. Auch dieser innengeführte Stempel kann innen nochmals hohl ausgeführt sein, um darin die axiale Einführung eines weiteren Instrumententeils zu erlauben, bei denen eine (nicht dargestellte) Setzschraube in das Gewinde an der Innenseite der Tulpenwände 11a, 11b eingeschraubt werden kann. Ebenfalls könnte im Instrument 200 ein (nicht dargestelltes) Schraubinstrument geführt werden, um die Pedikelschraube 30 bei vom Instrument 200 gehaltener Tulpe 10 in einen Wirbel einzuschrauben, im Falle der Ausgestaltungen mit Sattel 20 weist dieser hierzu eine den Schraubkopf der Pedikelschraube 30 zugänglich machende Zugangsöffnung auf.

Es versteht sich, dass über das Halteinstrument auch weitere Funktionen realisiert werden können, die dem Fachmann im Zusammenhang mit dem Einsetzen von Pedikelschrauben und Halten der Tulpen derartiger Wirbelsäulenimplantate vertraut sind.

## Patentansprüche

1. Instrumentensatz (10, 200), aufweisend eine sich entlang ihrer Längsachse (X) erstreckende Tulpe (10) eines eine Pedikelschraube (30) aufweisenden und der Ankopplung einer Verbindungsstange entlang einer Querachse (Q) der Tulpe (10) dienenden Wirbelsäulenimplantats (100) sowie eine Haltevorrichtung (200) zum temporären Halten der Tulpe (10) während eines Implantateinsatzvorgangs in einem Haltezustand des Instrumentensatzes, wobei ein Übergang vom Haltezustand in einen Freigabezustand von Tulpe (10) und Haltevorrichtung (200) und vice versa eine rotatorische Bewegung um eine Achse (A) beinhaltet, wobei die Achse (A) mit überwiegender Richtungskomponente quer zur Längsachse (X) und parallel zur Querachse (Q) der Tulpe verläuft,
**gekennzeichnet durch** eine
- tulpenseitig durch in Seitenwänden (11a, 11b) der Tulpe (10) gebildete kerbenartige Bereiche (12a, 12b) von wenigstens vier azimutal voneinander beabstandeten Kopplungsbereichen (12a, 12b, 18a, 18b) der Tulpe (10) zur Ankopplung der Haltevorrichtung im Haltezustand sowie
- haltevorrichtungsseitig durch radial von Innenseiten von Beinen (211) der Haltevorrichtung (200) vorragende Radialvorsprünge (212) gebildete Verdrehsperre (12a, 12b, 212a, 212b) gegen eine Verdrehung der Haltevorrichtung (200) gegenüber der Tulpe (10) um deren Längsachse (X) und durch eine
- tulpenseitig durch eine radial äußere Begrenzung eines axial an einen kerbenartigen Bereich anschließenden unteren Axialkanalbereichs (18a, 18b) sowie
- haltevorrichtungsseitig durch einen auf einem Radialvorsprung (212a, 212b) angeordneten und zur Aufnahme in den unteren Axialkanalbereich ausgelegten Axialvorsprung (218a, 218b) gebildete, die rotatorische Bewegung nach bezüglich der Tulpenlängsachse radial außen sperrende Rotationssperre (18a, 18b, 218a, 218b).

2. Instrumentensatz nach Anspruch 1, bei dem innerhalb des Übergangs eine Transition zwischen der bei bezüglich der Tulpenlängsachse (X) radial auf die Tulpe einwirkender Haltekraft bereits wirkenden Verdrehsperre (12a, 12b, 212a, 212b) der Rotationssperre (18a, 18b, 218a, 218b) erfolgt.

3. Instrumentensatz nach Anspruch 1 oder 2, bei dem der Übergang jedenfalls haltezustandsnah eine translatorische Axialbewegung zwischen Tulpe und Haltevorrichtung entlang der Tulpenlängsachse beinhaltet.

4. Instrumentensatz nach Anspruch 3, bei dem die rotatorische Bewegung und die Axialbewegung in dem Übergang wenigstens zeitweise überlappen.

5. Tulpe eines Instrumentensatzes gemäß einem der vorhergehenden Ansprüche.

6. Tulpe nach Anspruch 5, bei dem ein Boden (120) des kerbenartigen Bereichs in Grundform konkav verläuft.

7. Tulpe nach Anspruch 5 oder 6, mit mehreren in die obere distale Axialseite der Tulpenseitenwände mündenden oberen Axialkanalbereichen (18a, 18b), welche bevorzugt mit den unteren Kanalbereichen kommunizieren und insbesondere mit ihnen fluchten und in Form von in Axialrichtung verlaufenden Bohrungen (18a, 18b) gebildet sind, die azimutal als Langlöcher ausgebildet sind.

8. Tulpe nach einem der Ansprüche 5 bis 7, bei der zum axialen distalen Tulpenende weisende Seitenwände (122) der kerbenartigen Bereiche im Wesentlichen in einer Radialebene liegen, insbesondere auf gleicher axialer Höhe.

9. Tulpe nach einem der Ansprüche 5 bis 8, bei der eine zur proximalen axialen Endseite der Tulpe weisende Seitenwand (121) des kerbenartigen Bereichs rampenförmig gebildet ist.

10. Tulpe nach einem der Ansprüche 5 bis 9 mit einem azimutal zwischen zwei an einer Seitenwand der Tulpe ausgebildeten kerbenartigen Bereichen gebildeten Stegbereich, insbesondere mit radial nach außen abnehmender Azimutalabmessung.

11. Herstellung einer nach einem der Ansprüche 5 bis 10 gebildeten Tulpe, bei der die Ausbildung des unteren Axialkanalbereichs nach der Ausbildung des oberen Kanalbereichs erfolgt, und bei der bevorzugt die Ausbildung des unteren Kanalbereichs insbesondere vor der Ausbildung des kerbenartigen Bereichs und insbesondere vor Ausbildung der Tulpenseitenwände mittels Entfernung des zwischen den Seitenwänden gelegenen Materialbereichs erfolgt.

## Claims

1. An instrument set (10, 200), comprising a tulip (10), extending along a longitudinal axis (X), of a spinal implant (100) including a pedicle screw (30) and used to connect a connecting rod along a transverse axis (Q) of the tulip (10), and a retention device (200) for temporarily retaining the tulip (10) during an implantation process in a retained state of the instrument set, wherein a transition of the tulip (10) and retention device (200) from the retained state to a released state and vice versa involves a rotational movement around an axis (A), wherein the axis (A) runs with a predominant directional component transverse to the longitudinal axis (X) and parallel to the transverse axis (Q) of the tulip,
**characterised by** an anti-twist lock (12a, 12b, 212a, 212b) which acts against a twisting of the retention device (200) relative to the tulip (10) around the longitudinal axis (X) thereof, and which is formed
- on the tulip side, by notch-like regions (12a, 12b), formed in side walls (11a, 11b) of the tulip (10), of at least four coupling regions (12a, 12b, 18a, 18b) of the tulip (10) arranged azimuthally spaced apart from each other for coupling the retention device in the retained state, and
- on the retention device side, by radial projections (212) projecting radially from the inside of legs (211) of the retention device (200),
and by an anti-rotation lock (18a, 18b, 218a, 218b) which blocks the rotational movement radially outwards relative to the longitudinal axis of the tulip and which is formed
- on the tulip side, by a radially outer boundary of a lower axial channel region (18a, 18b) adjoining a notch-like region, and
- on the retention device side, by an axial projection (218a, 218b) arranged on a radial projection (212a, 212b) and configured for being received in the lower axial channel region.

2. The instrument set according to claim 1, wherein during the transition, a shift occurs between the anti-twist lock (12a, 12b, 212a, 212b) that is already acting while a retention force is exerted radially on the tulip relative to the longitudinal axis (X) of the tulip and the anti-rotation lock (18a, 18b, 218a, 218b).

3. The instrument set according to claim 1 or 2, wherein the transition involves a translational axial movement between the tulip and the retention device along the longitudinal axis of the tulip, at least near the retained state.

4. The instrument set according to claim 3, wherein the rotational movement and the axial movement overlap at least temporarily during the transition.

5. A tulip of an instrument set according to one of the preceding claims.

6. The tulip according to claim 5, wherein a base (120) of the notch-like region is concave in its basic form.

7. The tulip according to claim 5 or 6, including a plurality of upper axial channel regions (18a, 18b) opening into the upper distal axial side of the side walls of the tulip, said regions preferably communicating with the lower channel regions, and in particular being flush with the same and being configured in the form of boreholes (18a, 18b) extending in the axial direction and configured azimuthally as elongated holes.

8. The tulip according to one of claims 5 to 7, wherein side walls (122), facing the axial distal end of the tulip, of the notch-like regions substantially lie in a radial plane, in particular at the same axial height.

9. The tulip according to one of claims 5 to 8, wherein a side wall (121), facing the proximal axial end face of the tulip, of the notch-like region is configured in the form of a ramp.

10. The tulip according to one of claims 5 to 9, having a web region formed azimuthally between two notch-like regions formed in one side wall of the tulip, and in particular having an azimuthal dimension that decreases radially outwards.

11. Manufacture of a tulip configured according to claims 5 to 10, wherein the forming of the lower axial channel region takes place after the forming of the upper channel region, and wherein the forming of the lower channel region preferably takes place in particular before the forming of the notch-like region and in particular before the forming of the tulip side walls by removing the material region located between the side walls.

## Revendications

1. Ensemble d'instruments (10, 200) comprenant une tulipe (10), s'étendant le long d'un axe longitudinal (X), d'un implant rachidien (100) présentant une vis pédiculaire (30) et servant à coupler une tige de liaison le long d'un axe transversal (Q) de la tulipe (10), ainsi qu'un dispositif de retenue (200) permettant de retenir temporairement la tulipe (10) pendant une procédure d'insertion d'implant dans un état de retenue de l'ensemble d'instruments, un passage de l'état de retenue à un état de dégagement de la tulipe (10) et du dispositif de retenue (200) et vice versa faisant appel à un mouvement de rotation autour d'un axe (A), l'axe (A) ayant une composante directionnelle prédominante transversale à l'axe longitudinal (X) et parallèle à l'axe transversal (Q) de la tulipe,
**caractérisé par** un verrou anti-torsion (12a, 12b, 212a, 212b) et qui est destiné à empêcher une torsion du dispositif de retenue (200) par rapport à la tulipe (10) autour de l'axe longitudinal (X) de cette dernière qui est formé
- côté tulipe, par des zones en forme d'encoche (12a, 12b) qui font partie d'au moins quatre zones de couplage (12a, 12b, 18a, 18b) de la tulipe (10) espacées azimutalement pour coupler le dispositif de retenue en position de retenue et qui sont formées dans les parois latérales (11a, 11b) de la tulipe, ainsi que
- côté dispositif de retenue, par des protubérances radiales (212) faisant saillie radialement à partir des côtés intérieurs des pattes (211) du dispositif de retenue (200);
et par un verrou anti-rotation (18a, 18b, 218a, 218b) et qui bloque le mouvement de rotation radialement vers l'extérieur par rapport à l'axe longitudinal de la tulipe qui est formé
- côté tulipe, par une délimitation radialement extérieure d'une zone de canal axial inférieure (18a, 18b) dans la continuité axiale d'une zone en forme d'encoche, ainsi que
- côté dispositif de retenue, par une protubérance axiale (218a, 218b) agencée sur une protubérance radiale (212a, 212b) et conçue pour être reçue dans la zone de canal axial inférieure.

2. Ensemble d'instruments selon la revendication 1, dans lequel, dans le cadre du passage, il se produit une transition entre le verrou anti-torsion (12a, 12b, 212a, 212b) agissant déjà tandis qu'une force de retenue est appliquée radialement sur la tulipe par rapport à l'axe longitudinal (X) de cette dernière et le verrou anti-rotation (18a, 18b, 218a, 218b).

3. Ensemble d'instruments selon la revendication 1 ou 2, dans lequel le passage fait appel, au moins près de l'état de maintien, à un mouvement axial de translation entre la tulipe et le dispositif de retenue le long de l'axe longitudinal de la tulipe.

4. Ensemble d'instruments selon la revendication 3, dans lequel le mouvement de rotation et le mouvement axial coïncident au moins temporairement lors du passage.

5. Tulipe d'un ensemble d'instruments selon l'une des revendications précédentes.

6. Tulipe selon la revendication 5, dans laquelle le fond (120) de la zone en forme d'encoche est concave dans sa forme de base.

7. Tulipe selon la revendication 5 ou 6, comportant plusieurs zones de canal axial supérieures (18a, 18b) débouchant dans le côté axial distal supérieur des parois latérales de la tulipe, lesquelles zones communiquent de préférence avec les zones de canal inférieures et sont notamment de niveau avec celles-ci et sont constituées sous la forme d'alésages (18a, 18b) qui s'étendent dans la direction axiale et sont conçus azimutalement sous la forme de trous allongés.

8. Tulipe selon l'une des revendications 5 à 7, dans laquelle des parois latérales (122) des zones en forme d'encoche, lesquelles parois sont tournées vers l'extrémité distale axiale de la tulipe, se trouvent sensiblement dans un plan radial, notamment à la même hauteur axiale.

9. Tulipe selon l'une des revendications 5 à 8, dans laquelle une paroi latérale (121) de la zone en forme d'encoche, laquelle paroi est tournée vers le côté terminal axial proximal de la tulipe, est constituée en forme de rampe.

10. Tulipe selon l'une des revendications 5 à 9, comportant une zone d'âme qui est formée azimutalement entre deux zones en forme d'encoche constituées au niveau d'une paroi latérale de la tulipe et qui présente notamment une dimension azimutale diminuant radialement vers l'extérieur.

11. Production d'une tulipe formée selon l'une des revendications 5 à 10, dans laquelle la constitution de la zone de canal axial inférieure a lieu après la constitution de la zone de canal supérieure, et dans laquelle de préférence la constitution de la zone de canal inférieure a lieu notamment avant la constitution de la zone en forme d'encoche et notamment avant la constitution des parois latérales de la tulipe par enlèvement de la zone de matériau située entre les parois latérales.
